# EUROPÄISCHE PATENTANMELDUNG

(11) **EP 2 036 583 A2**
(43) Veröffentlichungstag der Anmeldung: **18.03.2009**
(21) Anmeldenummer: 08161271.5
(22) Anmeldetag: 28.07.2008
(51) Int. Cl.: A61L 31/02, A61L 31/16

(54) **Stent mit einer Beschichtung**

(30) Priorität: 14.09.2007 DE 102007043883
(71) Anmelder: BIOTRONIK VI Patent AG, 6341 Baar (CH)
(72) Erfinder: Adden, Nina, 90427, Nürnberg (DE); Borck, Alexander, 91086, Aurachtal (DE)
(74) Vertreter: Lindner-Vogt, Karin L.

(57) **Zusammenfassung**

Die Erfindung geht aus von einem Stent mit einer Beschichtung. Die erfindungsgemäße Beschichtung besteht aus oder enthält
(i) L-3,4-Dihydroxyphenylalanin (L-DOPA) oder
(ii) ein Derivat von L-3,4-Dihydroxyphenylalanin (L-DOPA), das durch ionische oder kovalente Anbindung eines pharmazeutischen Wirkstoffs an die Amino- oder Säurefunktion von L-3,4-Dihydroxyphenylalanin (L-DOPA) erhältlich ist.

## Beschreibung

Die Erfindung betrifft einen Stent mit einer Beschichtung.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatwerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe, aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden.

Trotz der erreichten Fortschritte besteht noch immer ein hohes Bedürfnis, eine bessere Integration des Stents in sein biologisches Umfeld zu erreichen und dadurch die Restenoserate zu senken.

### Erfindungsgemäße Lösung

Die Erfindung geht aus von einem Stent mit einer Beschichtung. Die erfindungsgemäße Beschichtung besteht aus oder enthält
(i) L-3,4-Dihydroxyphenylalanin (L-DOPA) oder
(ii) ein Derivat von L-3,4-Dihydroxyphenylalanin (L-DOPA), das durch ionische oder kovalente Anbindung eines pharmazeutischen Wirkstoffs an die Amino- oder Säurefunktion von L-3,4-Dihydroxyphenylalanin (L-DOPA) erhältlich ist.

Der Erfindung liegt die Erkenntnis zugrunde, dass L-DOPA (IUPAC-Name: (2S)-2-Amino-3-(3,4-dihydroxyphenyl)propansäure) und seine Derivate ein besonders hohes Haftvermögen auf gängigen Implantatwerkstoffen besitzen und damit den spezifischen Anforderungen an Stents in besonderer Weise genügen, deren Oberfläche bei zweckbestimmter Anwendung hohen mechanischen Kräften ausgesetzt sind. L-DOPA ist bekanntermaßen eine Hauptkomponente des Klebstoffes, mit dem sich Miesmuscheln auf festen Oberflächen festkleben. Die Anbindung erfolgt vornehmlich über die beiden OH-Gruppen, und daher führt insbesondere eine Kombination von L-DOPA und seinen Derivaten mit Grundkörpern von Stents, die eine polare Oberfläche aufweisen, zu Beschichtungen mit einem sehr hohen Haftvermögen. Im Gegensatz zu gängigen Monoschichten wie zum Beispiel Silanschichten kann L-DOPA nicht nur auf Metall binden, sondern auch relativ unspezifisch fast an alle Oberflächen anbinden.

L-DOPA ist eine nicht-proteinogene □-Aminosäure, die im Körper aus Tyrosin mit Hilfe des Enzyms Tyrosinhydroxylase gebildet wird. Die Verbindung wird unter dem Namen Levodopa als Arzneimittel eingesetzt. Bei bestimmungsgemäßen Gebrauch als Beschichtungsmaterial für Stents ist daher mit einer hohen Biokompatibilität zu rechnen.

L-DOPA weist einen 2-Aminopropansäurerest auf, über dessen beide Funktionalitäten, pharmazeutisch wirksame Verbindungen angebunden werden können. Sind die eingesetzten Wirkstoffe Säuren, Basen oder Salze, so kann die Anbindung auf ionischer Wechselwirkung mit den beiden Funktionalitäten des L-DOPA beruhen. Alternativ ist eine kovalente Anbindung des Wirkstoffs über die Säure- oder Aminofunktion möglich. Die kovalente Bindung kann derart beschaffen sein, dass eine Spaltung und damit wieder kontrollierte Freisetzung des Wirkstoffs im Körper möglich ist. Der Wirkstoff wird demnach im Sinne eines Pro-Pharmakons mit der Implantation eingebracht und wird erst im Organismus in die Wirkform umgewandelt. Denkbar ist jedoch auch, dass die kovalente Anbindung in vivo bestehen bleibt und der Wirkstoff auf der Implantatoberfläche immobilisiert ist. L-Dopa und seine Derivate sind neben den metallischen Oberflächen für eine Vielzahl von Oberflächen geeignet. Der Vorteil der Methode liegt im unspezifischen Bindungsvermögen.

Die Herstellung eines Stents mit einer Beschichtung, die solch ein Derivat von L-DOPA enthält oder aus solch einem Derivat von L-DOPA besteht, kann vorzugsweise über einen Stent erfolgen, der eine Beschichtung aufweist, die aus L-DOPA besteht oder dieses enthält. Mit anderen Worten, zunächst wird ein mit L-DOPA beschichteter Stent hergestellt. Anschließend erfolgt die ionische oder kovalente Anbindung des Wirkstoffs über das auf der Implantatoberfläche immobilisierte L-DOPA.

Als Wirkstoffe können beispielsweise Aptamere, RGD-Sequenzen, Antikörper, wie CD 133, Antikoagulantien, Proliferationshemmer, Entzündungshemmer, Calciumkanalblocker und Entothelinrezeptor-Antagonisten und andere verwendet werden. Bevorzugte Wirkstoffe umfassen insbesondere cRGD, dODN's, Bosentan, Antisence, Sirolimus und dessen Derivate (wie Biolimus und Everolimus) sowie Pimecrolimus.

Aptamere sind kurze einzelsträngige DNA- oder RNA-Oligonukleotide (25-70 Basen), die ein spezifisches Molekül über ihre 3D-Struktur binden können. Sie binden an Proteine, zum Beispiel Wachstumsfaktoren und bakterielle Gifte, niedermolekulare Stoffe, wie Aminosäuren und Antibiotika, und auch an Viruspartikel. Aptamere zeichnen sich durch hohe Spezifität und Affinität, hohe
chemische Stabilität, niedrige Immunogenizität und die Fähigkeit der gezielten Beeinflussung von Protein-Protein-Interaktionen aus. Aptamere und insbesondere Spiegelmere sind durch ihre Konformation vor DNAsen (Enzyme die DNA abbauen) geschützt und besitzen eine ausreichend hohe Halbwertszeit im Körper.

Eine RGD-Sequenz ist eine Aminosäuresequenz aus den drei Aminosäuren Arginin, Glycin und Asparaginsäure, kurz Arg-Gly-Asp, oder im Einbuchstaben-Code RGD. Die RGD-Sequenz kommt besonders in Proteinen der Extrazellulären Matrix (Bindegewebe) vor, zum Beispiel in Fibronektin und Vitronektin. Zellen können mit Hilfe von bestimmten Zelloberflächen-Rezeptoren, den Integrinen, an die RGD-Sequenz binden. Die RGDvermittelte Zelladhäsion dient zur mechanischen Verankerung von Zellen. Implantate können zur besseren Integration in den Körper mit der RGD-Sequenz beschichtet werden. Die RGD-Sequenz wird in den unterschiedlichen Matrixproteinen in unterschiedlicher Konformation präsentiert, welche zum Teil spezifisch von den Integrin-Subtypen erkannt wird (zum Beispiel cyclische RGD-Peptide, wie Cilengitide). Die cRGD besitzen eine Konformation, die es ihnen ermöglicht, selektiv an Integrine von bestimmten Zellen zu koppeln. Dabei kann zwischen SMC- und EC-Zellen unterschieden werden. Durch das binden an die Bindungsdomäne der Intergins kann ein Adherieren erzwungen oder verhindert werden.

Antikörper (Immunglobuline) sind Proteine aus der Klasse der Globuline, die in Wirbeltieren als Reaktion auf bestimmte eingedrungene Fremdstoffe, als Antigene bezeichnet, gebildet werden. Sie dienen der Abwehr dieser Fremdstoffe. Ein bestimmtes Antigen induziert in der Regel die Bildung nur eines bestimmten, dazu passenden Antikörpers, der spezifisch nur an diesen Fremdstoff gebunden wird. Bevorzugt ist der Einsatz der Antikörper CD 133 und CD 34.

Vorzugsweise weist der Stent einen metallischen Grundkörper auf. Insbesondere besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient beispielsweise künstliches Plasma, wie es nach EN ISO 10993-15:2000 für Biokorrosionsuntersuchungen vorgeschrieben ist (Zusammensetzung NaCI 6,8 g/l, CaCI₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf ihr Korrosionsverhalten hin untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Nachfolgend wird die Erfindung anhand eines Ausführungsbeispiels näher erläutert.

Ein Stent aus der biokorrodierbaren Magnesiumlegierung WE43 (nach ASTM) wird entfettet und getrocknet. Die Beschichtung wird wie folgt durchgeführt:

Der Stent wird bei einem pH-Wert 8 bis 10 in PBS-Puffer für 10 Minuten bis zu einer Stunde in eine 2%ige L-DOPA-Lösung getaucht, anschließend wieder entnommen, mit Reinstwasser gewaschen und getrocknet.

Die funktionalisierten Stents werden für 3 bis 5 Stunden in N,N'-Carbonyldiimidazol (CDI) gegeben. Dazu wird das CDI in trocknem Dioxan gelöst. Hier eignet sich eine Stammlösung von 2,5 g / 50 ml CDI in Dioxan, die für mehrere Tage (2, trocken) haltbar ist. Die Stents werden bei Raumtemperatur leicht bewegt.

Nach der Aktivierung werden die Stents entnommen und mit trocknem Dioxan gespült.

Für die Ankopplung der Antikörper werden die aktivierten Stents in die Antikörper-Lösung getaucht und bei 4°C über Nacht (min. 12 Std.) gekoppelt.
Die Reaktion geschieht am geeignetsten in 125 mM Natriumborat mit 0,066% SDS bei einem pH-Wert von 10.

Die Lösung ist danach wiederverwendbar bzw. es können mehrere Oberflächen mit dieser Lösung behandelt werden.

Die Stents werden nach der Kopplung dreimal mit 5 ml des Borax-Puffers (oben) und danach dreimal mit Wasser gewaschen. Die nach diesen Waschschritten noch analysierbaren Antikörper sind kovalent gebunden.

## Patentansprüche

1. Stent mit einer Beschichtung, **dadurch gekennzeichnet, dass** die Beschichtung
(i) L-3,4-Dihydroxyphenylalanin (L-DOPA) oder
(ii) ein Derivat von L-3,4-Dihydroxyphenylalanin (L-DOPA), das durch ionische oder kovalente Anbindung eines pharmazeutischen Wirkstoffs an die Amino- oder Säurefunktion von L-3,4-Dihydroxyphenylalanin (L-DOPA) erhältlich ist,
enthält oder hieraus besteht.

2. Stent nach Anspruch 1, bei dem der Stent einen metallischen Grundkörper aufweist, der die Beschichtung trägt.

3. Stent nach Anspruch 1 oder 2, bei dem der metallische Grundkörper des Stents aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung besteht.
